# EUROPEAN PATENT SPECIFICATION

(11) **EP 2 554 176 B1**
(45) Date of publication and mention of the grant of the patent: **27.07.2016**
(21) Application number: 11006409.4
(22) Date of filing: 04.08.2011
(51) Int. Cl.: A61K 35/28, A61P 1/16

(54) **Means for liver regeneration**
Mittel zur Leberregeneration
Supports de régénération de foie

(43) Date of publication of application: 06.02.2013
(73) Proprietor: ETHIANUM Betriebsgesellschaft mbH & Co. KG, 69115 Heidelberg (DE); Hopp Stiftung GmbH, 69190 Walldorf (DE)
(72) Inventor: Germann, Günter, 69115 Heidelberg (DE); Köllensperger, Eva, 67227 Frankenthal (DE)
(74) Representative: Patent- und Rechtsanwälte Ullrich & Naumann

(56) References cited:
- WO-A2-2007/127698
- WO-A2-2010/070141
- PARKER A M ET AL: "Adipose-derived stem cells for the regeneration of damaged tissues", EXPERT OPINION ON BIOLOGICAL THERAPY, ASHLEY, LONDON, GB, vol. 6, no. 6, 1 January 2006 (2006-01-01) , pages 567-578, XP009089525, ISSN: 1471-2598
- GHAEDI MAHBOOBE ET AL: "Bottom-up signaling from HGF-containing surfaces promotes hepatic differentiation of mesenchymal stem cells", BIOCHEMICAL AND BIOPHYSICAL RESEARCH COMMUNICATIONS, vol. 407, no. 2, April 2011 (2011-04), pages 295-300, XP009155342,
- THOLPADY S S ET AL: "Adipose stem cells and solid organ transplantation", CURRENT OPINION IN ORGAN TRANSPLANTATION 2009 LIPPINCOTT WILLIAMS AND WILKINS USA LNKD- DOI:10.1097/MOT.0B013E328320D2CF, vol. 14, no. 1, February 2009 (2009-02), pages 51-55, XP009155341, ISSN: 1087-2418
- BANAS AGNIESZKA ET AL: "Rapid hepatic fate specification of adipose-derived stem cells and their therapeutic potential for liver failure.", JOURNAL OF GASTROENTEROLOGY AND HEPATOLOGY JAN 2009, vol. 24, no. 1, January 2009 (2009-01), pages 70-77, ISSN: 1440-1746

## Description

The present invention pertains to means for the treatment of acute and chronic liver disease. In particular, it relates to a composition comprising human adipose stem cells for the prevention, amelioration or treatment of acute or chronic liver disease. Further encompassed by the disclosure is a kit comprising said composition and optionally means for isolating adipose stem cells and/or means for administering adipose stem cells.

Chronic liver disease is marked by the gradual destruction of liver tissue over time. Several liver diseases fall under this category, including cirrhosis and fibrosis, the latter of which is often the precursor of cirrhosis.

Cirrhosis is the result of acute and chronic liver disease and is characterized by the replacement of liver tissue by fibrotic scar tissue and regenerative nodules leading to a progressive loss of liver function. Fibrosis and nodular regeneration results in the loss of the normal microscopic lobular architecture of the liver. Fibrosis represents the growth of scar tissue resulting from, for example, infection, inflammation, injury, and even healing. Over time, the fibrotic scar tissue slowly replaces the normal functional liver tissue resulting in a decreasing amount of blood flow to the liver leaving the liver incapable of fully processing nutrients, hormones, drugs, and poisons that are found in the blood stream. More common causes of cirrhosis include alcoholism, hepatitis C viral infections, ingestion of toxins, and fatty liver, but many other possible causes also exist.

Chronic hepatitis C virus infection and non-alcoholic steatohepatitis are the two major causes of chronic liver disease in the United States estimated to affect between three and five million people. A rising concern is the continuously increasing number of US citizens, currently numbering over 30 million, with obesity and metabolic syndrome that have non-alcoholic fatty liver disease with approximately 10 percent who will eventually develop non-alcoholic steatohepatitis. Other bodily complications are a consequence of a loss of liver function. The most common complication of cirrhosis is a condition known as ascites, an accumulation of fluid in the peritoneal cavity, which can lead to an increased risk of spontaneous bacterial peritonitis possibly resulting in the premature death of the patient. Other potentially life-threatening complications of cirrhosis include hepatic encephalopathy, a neuropsychotic abnormality resulting when toxic substances that normally are removed by the liver from blood begin to impede proper functioning of brain cells. Yet another potentially life-threatening complication of cirrhosis includes esophageal viruses or extremely dilated sub-mucosal veins in the esophagus that are susceptible to bleeding.

Once any cirrhosis or fibrosis has occurred in the liver, it is generally considered irreversible. Rather, conventional treatment focuses on preventing any further progression of cirrhosis in the liver and mitigating the complications that can arise from cirrhosis. In more advanced stages of cirrhosis, the only conventionally known treatment is a liver transplant. The American Liver Foundation estimates that over 300,000 people in the United States are hospitalized each year as a result of cirrhosis of the liver. It is also estimated that 18,000 people are in need of liver transplants. In Germany, the number of liver transplantations was 5,083 in 2010, whereas about 2,000 patients are in need of liver transplants.

Liver cell transplantation is an emerging procedure, involving the infusion of liver cell suspension in the portal system of the recipient. It aims to a recovery of the recipient's liver function as a consequence of engraftment and repopulation of the diseased parenchyma. However, because supply of mature human hepatocytes for transplantation is still limited, in fact more or less as limited as availability of whole liver, research also aims at obtaining transplantable cells from other sources, such as progenitor and stem cells, e.g. of embryonic or adult origin, that could be expandable, e.g. *in vitro,* and able to differentiate into functional mature hepatocytes, especially *in vivo* after transplantation. Accordingly, there is a great need to develop means that are useful in treating various diseases or conditions associated with liver-associated diseases, particularly given the inadequate treatments currently available for the majority of these disorders.

Thus, the technical problem underlying the present invention could be seen as the provision of means and methods which comply with the aforementioned needs. This technical problem has been solved by the embodiments characterized in the claims and herein below.

The inventors' aim of this study was to evaluate the possibility of stem cell transplantation in order to regenerate liver, in particular in the field of chronic liver diseases, broaden the currently limited therapeutic options, and consequently to improve the quality of life and overall survival of affected patients. Therefore, the integration of adipose stem cells into diseased liver tissue and their ability to take over liver specific functions was analyzed. Accordingly, a chronic liver damage with predominantly periportal location, a pattern comparable to that seen in viral hepatitis, which is the most frequent cause of chronic liver failure [Jung et al. 2000, Scand J Gastroenterol 35, 969] was established in rats.

For that purpose allylalcohol was used which induces a predominantly periportal damage with a peak occurring 48 hours after injection. A high variance in the degree of necrosis from rat to rat, lobe to lobe and section to section could be observed in this experiment which is consistent with the results of other studies using the rat model system. However, the inventors could show with their experiments that the injection of adipose stem cells into the damaged liver after 2/3 hepatectomy leads to a significantly higher and earlier restoration of liver function compared with the non cell treated control group. This could be demonstrated by higher albumin and total protein levels. Importantly, the inventors did not observe any negative side effects of cell therapy, especially no tumor formation.

Strikingly, injected stem cells could be found from week 1 up to more than week 8 post surgery in histological sections migrating from the center of the lobe to the periphery. No clearing reactions of dead stem cells by macrophages were seen and no superparamagnetic iron oxide particles (SPIO) particles could be found in macrophages.

Also, in this experiment, the lower iron levels in cell treated animals 3 weeks post surgery can be interpreted as faster regeneration due to the injected stem cells. Furthermore, it makes a high turnover of the SPIO-marked stem cells unlikely as this would be expected to lead to an increase of iron levels.

Finally, it has been found by the inventors, that the way of administration of MSCs to the liver does have an impact on their distribution and function in liver parenchyma. In this study, the adipose stem cells injected directly into the remaining lateral hepatic lobe migrated from an initially central location within the lobe to its periphery and could be found up to 8 weeks after injection. This observation is indeed astonishing, since in other studies stem cells were injected via the tail vein of mice (Banas et al. 2009, Hepatology 24, 70) or into the spleen (Aurich et al. 2009, Gut 58, 570), however, these studies do not report as successful results as those made by the present inventors. All the more, even a clinical study in which adipose tissue derived stromal cells were aministered by intrahepatic arterial was suspended (http://clinicaltrials.gov/ct2/show/NCT01062750; Clinical Trials.gov Identiffier: NTC01062750). Hence, it is apparent that the way of administration is important for a successful implantation of stem cells, in particular adipose stem cells.

WO 200727698 discloses transplantation of adipose stem cells via intrahepatic injection after encapsulation.

It must be noted that as used herein, the singular forms "a", "an", and "the", include plural references unless the context clearly indicates otherwise. Thus, for example, reference to "an antibody" includes one or more of such different antibodies and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim. In each instance herein any of the terms "comprising", "consisting essentially of" and "consisting of" may be replaced with either of the other two terms.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or" as used herein.

Several documents are cited throughout the text of this specification. Each of the documents cited herein (including all patents, patent applications, scientific publications, manufacturer's specifications, instructions, etc.), whether *supra* or *infra,* are hereby incorporated by reference in their entirety. Nothing herein is to be construed as an admission that the invention is not entitled to antedate such disclosure by virtue of prior invention.

Given the findings of the present inventors, the present invention relates a comprising human adipose stem cells for regeneration of liver (including liver tissue and/or liver cell regeneration). As used herein, when adipose stem cells are applied "for", e.g., regeneration of liver, it is meant that adipose stem cells are "for use in a method for", e.g., liver regeneration. Accordingly, the present invention also relates to a method of regeneration of liver in a subject in need thereof comprising administering adipose stem cells to said subject, preferably in an amount which is sufficient to regenerate liver. Due to medical conditions damaging liver, in particular those described herein in the context of chronic or acute liver disease, a need arises for regeneration of liver. Damage of liver may be caused by any of the medical conditions described herein in the context of acute or chronic liver disease. Regeneration of liver is, however, also important after liver surgery (including liver resectomy, in particular partial liver resectomy), for example, when a liver tumor is removed. Not only by the successful implantation of stem cells into damaged liver, but also by resecting 2/3 of the liver the present inventors demonstrated that the composition of the present invention is useful for the regeneration of liver.

The term "liver" when used herein includes the whole organ liver or parts thereof, liver tissue and/or liver cells.

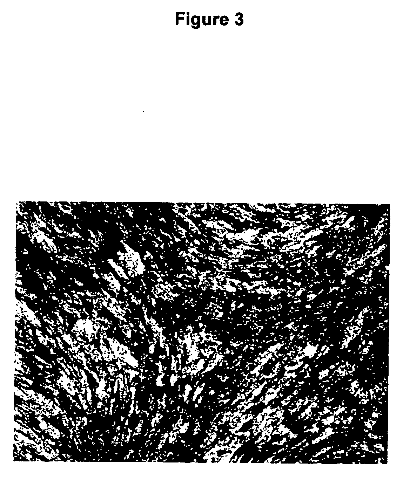

When used herein "regeneration of liver" includes *de novo* generation of liver cells, liver tissue and the whole organ liver from adipose stem cells. "Regeneration of liver" also includes activation/stimulation of liver cells that are able to repair liver structure and/or to repopulate liver parenchyma. Regeneration of liver can preferably be determined/observed by the function of liver, in particular by measuring the various liver parameters described herein such as those described in Example 6.4 and/or by analyzing the expression of hepatocyte-specific RNAs such as AFP (alpha-fetoprotein), CK19 (cytokeratin), CK7, CX43 (connexin) (typical for early differentiated hepatocytes) and/or CYP3A4 (cytochroime), PCK1 (phosphoenolpyruvate carboxykinase), CPS (caramoyl phosphate synthetase), CK18, CX32, CD26, ALB, TFN (transferrin) (typical for late differentiated hepatocytes); see Figure 3 in Aurich et al. 2009, Gut 58, 570). Alternatively or in addition, regeneration of liver can be assessed by histology of a liver specimen which can be obtained, for example, by biopsy. Also, alternatively or in addition, liver regeneration can be assessed by immunofluorescence of glycogen or CK19.

In a preferred aspect, the present invention relates to a composition comprising human adipose stem cells for the prevention, amelioration or treatment of acute or chronic liver disease. Said composition is thus for use in the prevention, amelioration or treatment of acute or chronic liver disease. Also, said composition is for use in a method for the prevention, amelioration or treatment of acute or chronic liver disease comprising administering said composition to a subject in need thereof.

The term "composition" as used herein means a composition which comprises human adipose stem cells which can be used for the prevention, amelioration, or treatment of acute or chronic liver disease. Thus, in one preferred embodiment of the present invention, the composition of the invention is intended for therapeutic applications, e.g. for tissue engineering and cell therapy. A skilled person will appreciate that the herein detailed composition may involve the use of the progenitor of the human adipose stem cells, the human adipose stem cells in their non-differentiated or in differentiated form, genetically modified human adipose stem cells, cell lines thereof, cell populations comprising such, as well as the progeny thereof, including the differentiated or non-differentiated progeny or genetically modified derivatives thereof. As noted above, these cells can be used, e.g., for cell replacement therapies, in which the diseased liver is engrafted and repopulated by the adipose stem cells to recover the recipient's liver function. The cells can be administered to a tissue of interest, preferably liver tissue, in a subject to supplement functioning cells or replace cells, which have lost function. Preferably, said subject is a human. Alternatively, differentiated human adipose stem cells are also contemplated, wherein human adipose stem cells are isolated, differentiated in the presence of the differentiation and/or growth factors *in vitro,* and administered to a subject, as detailed herein. Diseased states or deficiencies typified by loss of liver mass and/or function, and that could benefit from human adipose stem cells as described herein include acute or chronic liver (or hepatic) diseases.

The term "stem cell" as used herein refers to a progenitor cell capable of self-renewal, i.e., which can proliferate without differentiation, whereby the progeny of a stem cell or at least part thereof substantially retains the unspecialized or relatively less specialized phenotype, the differentiation potential, and the proliferation competence of the mother stem cell. The term encompasses stem cells capable of substantially unlimited self-renewal, i.e., wherein the capacity of the progeny or part thereof for further proliferation is not substantially reduced compared to the mother cell, as well as stem cells which display limited self-renewal, i.e., wherein the capacity of the progeny or part thereof for further proliferation is demonstrably reduced compared to the mother cell.

A skilled person knows that the above properties generally refer to the *in vivo* behavior of progenitor and stem cells, and may under appropriate conditions be completely or at least in part replicated *in vitro* and/or ex *vivo.*

Based on the ability to give rise to diverse cell types, a progenitor or stem cell may be usually described as totipotent, pluripotent, multipotent or unipotent. A single "totipotent" cell is defined as being capable of growing, i.e. developing, into an entire organism. A "pluripotent" stem cell is not able of growing into an entire organism, but is capable of giving rise to cell types originating from all three germ layers, i.e., mesoderm, endoderm, and ectoderm, and may be capable of giving rise to all cell types of an organism. A "multipotent" cell is capable of giving rise to at least one cell type from each of two or more different organs or tissues of an organism, wherein the said cell types may originate from the same or from different germ layers, but is not capable of giving rise to all cell types of an organism. An "unipotent" cell is capable of differentiating to cells of only one cell lineage.

Accordingly, the term "adipose stem cell" as used herein denotes a multipotent cell type originally derived from adipose tissue. The person skilled in the art will appreciate that the herein detailed composition may involve the use of the progenitor of the human adipose stem cells, the human adipose stem cells in their non-differentiated form or in a differentiated form, genetically modified human adipose stem cells, cell lines thereof, cell populations comprising such, as well as the progeny thereof, including the differentiated or non-differentiated progeny or genetically modified derivatives thereof. For example, the adipose stem cells include, without being limited thereto, adipose-derived vascular-stromal cells, which are precursor/progenitor cells. Adipose stem cells also include preadipocytes or adipose-derived interstitial cells, adipose-derived stem cells, fat stem cells, endothelial progenitor cells, hematopoietic stem cells or mesenchymal stem cells. The term "adipose tissue" as used herein means adipose tissue or body fat or fat depot which is loose connective tissue composed of, inter alia, adipocytes and adipose stem cells.

Without being bound by theory, it is assumed that stem cells from adipose tissue become preadipocytes which then become adipocytes. Accordingly, adipose tissue comprises stem cells, preadipocytes and/or adipocytes. It may also comprise lipoblasts. In humans, adipose tissue is located, for instance, beneath the skin, around internal organs, in bone marrow and in breast tissue.

Adipose tissue is found in specific locations, which are referred to as "adipose depots". Adipose tissue contains several cell types, with the highest percentage of cells being adipocytes which contain fat droplets. Other cell types include fibroblasts, macrophages, endothelia cells and adipose stem cells. Adipose stem cells may be prepared and cultured according to conventional methods. For example, adipose stem cells may be isolated by mechanical mincing/grinding of, for example, fat lobes and/or fat tissue, followed by enzymatic digestion such as collagenase. Fat tissue or fat lobes can, for example, be isolated and/or separated from adipose tissues by means of liposuction, precipitation, treatment with an enzyme, such as collagenase, and removal of the supernatant such as red blood cells via centrifugation, as described, e.g., in WO 2005/042730. The isolation from adipose tissues, cultivation and differentiation of adipose stem cells are also shown in the following examples. It is further preferred, that the adipose stem cell as defined herein is a human adipose stem cell, i.e. derived from a human being. The adipose stem cells in the composition or kit of the invention may be used alone or in combination with other mesenchymal stem cells, such as, e.g. endothelial precursor cells.

As used herein, the term "isolated cell" refers generally to a cell that is not associated with one or more cells or one or more cellular components with which the cell is associated *in vivo.* For example, an isolated cell may have been removed from its native environment, or may result from propagation, e.g., *ex vivo* propagation, of a cell that has been removed from its native environment. Preferably, the adipose stem cell as used herein is an isolated adipose stem cell. For instance, adipose stem cells may be isolated from adipose tissue, for example, subcutaneous or peritoneal adipose tissue, or other suitable tissue comprising adipose stem cells. Preferably, the adipose stem cells are isolated from a human.

The term *"in vitro"* as used herein denotes outside, or external to, animal or, preferably, human body. The term *"in vitro"* as used herein should be understood to include "ex *vivo".* The term "ex *vivo"* typically refers to tissues or cells removed from an animal or human body and maintained or propagated outside the body, e.g., in a culture vessel.

The term "cell population" as used herein refers generally to a grouping of cells. Unless indicated otherwise, the term refers to a cell grouping consisting of or comprising isolated adipose stem cells as defined herein. A cell population may consist of cells having a common phenotype or may comprise at least a fraction of cells having a common phenotype. Cells are said to have a common phenotype when they are substantially similar or identical in one or more demonstrable characteristics, including but not limited to morphological appearance, the presence, absence or level of expression of particular cellular components or products, e.g., RNA, proteins, cell-specific markers or other substances, activity of certain biochemical pathways, proliferation capacity and/or kinetics, differentiation potential and/or response to differentiation signals or behavior during *in vitro* cultivation, e.g., adherence, non-adherence, monolayer growth, proliferation kinetics or the like. Such demonstrable characteristics may therefore define a cell population or a fraction thereof.

When a cell population is said herein to be "heterogeneous", this generally denotes a cell population comprising two or more cells or fractions of cells not having a common phenotoype, e.g., a cell population comprising cells of two or more different cell types. By means of example and not limitation, a heterogeneous cell population can be isolated from adipose tissue or body fat or fat depot and may comprise divers cell types including but not limited to adipose stem cells, fibroblasts, macrophages, and endothelial cells.

When a cell population is said herein to be homogenous, it consists of cells having a common phenotype. A cell population said herein to be "substantially homogeneous" comprises a substantial majority of cells having a common phenotype. A "substantially homogeneous" cell population may comprise at least 70 %, at least 80 %, preferably at least 90 %, at least 95 % or even at least 99 % of cells having a common phenotype such as the phenotype specifically referred to, e.g. the phenotype of human adipose stem cells as defined herein or progenitors of human adipose stem cells as defined herein. As used herein, the term "substantially homogeneous" does also encompass a homogeneous population.

The term "cell population comprising human adipose stem cells or progenitors thereof" refers to a cell population as defined herein comprising at least one progenitor or human adipose stem cell and typically a fraction of progenitor cells or human adipose stem cells, as defined herein. Usually, the progenitor or human adipose stem cells of the said fraction may have a common phenotype.

The term "progenitor cell" refers generally to an unspecialized or relatively less specialized and proliferation-competent cell, which or the progeny of which can give rise to at least one relatively more specialized cell type. By means of example and not limitation, a progenitor cell may give rise to descendents that can differentiate along one or more lineages to produce increasingly relatively more specialized cells, wherein such descendents and/or increasingly relatively more specialized cells made themselves be progenitor cells, or even to produce terminally differentiated cells, i.e. fully specialized cells, which may be post-mitotic. The term also encompasses stem cells as defined herein.

A progenitor cell is said to "give rise" to another, relatively more specialized cell when, by means of example and not limitation, the progenitor cell differentiates to become the other cell without first undergoing cell division, or the other cell is produced after one or more rounds of cell division and/or differentiation of the progenitor cell or progeny thereof.

The term "treat", "treating", or "treatment" as used herein denotes the improvement or even elimination of one or more symptoms associated with an acute or chronic liver disease as defined herein, by the administration of a composition of the invention comprising human adipose stem cells to a subject in need thereof or by using the kit of the invention.

The term "amelioration" as used herein refers to any improvement of the disease state of a patient having an acute or chronic liver disease specified herein, by the administration of a composition of the invention comprising human adipose stem cells to a subject in need thereof or by using the kit of the invention. Such an improvement may also be seen as a slowing or stopping of the progression of the disease of a patient having an acute or chronic liver disease set forth herein.

The term "prevention" as used herein means the avoidance of the occurrence or re-occurrence of a liver disease as specified herein, by the administration of a composition of the invention comprising human adipose stem cells to a subject in need thereof or by using the kit of the invention.

By using the composition or kit of the invention comprising the adipose stem cells described herein, regeneration of the liver, accelerated healing after partial resection of the liver, accelerated or increased recovery of the liver function in liver insufficiency, or a reduced risk of occurrence of hepatic dysfunctions or hepatic impairment or a reduced effect of metabolic diseases can advantageously be achieved. At the same time, adverse effects are minimized, in comparison to conventional therapies, such as liver transplantation.

The term "liver disease" as used herein refers to a hepatic disorder. Generally, a liver disease may be caused by any condition that results in the disturbance of the morphological and/or functional integrity of a body's liver. The etiology and treatment of liver diseases are described, e.g., in Oxford Textbook of Medicine (Warrell, Oxford Textbook of Medicine) David A. Warrell, Timothy M. Cox, John D. Firth, Oxford University Press, USA; 5 edition (July 22, 2010) and Johns Hopkins Internal Medicine Board Review 2010-2011: Certification and Recertification: Expert Consult - Online and Print (Redonda Miller, Bimal Ashar MD, Stephen Sisson, Johns Hopkins Hospital Mosby; 3 edition (March 2, 2010).

The term "acute liver disease" as used herein means the appearance of severe complications rapidly after the first signs of liver disease, such as jaundice, and indicates that the liver has sustained damage. The complications are, e.g., hepatic encephalopathy and impaired protein synthesis, as measured, for instance, by the levels of serum albumin and the prothrombin time in the blood. The 1993 classification defines hyperacute as within 1 week, acute as 8-28 days and subacute as 4-12 weeks (Williams et al. 1993, Lancet 342, 273). It reflects the fact that the pace of disease evolution strongly influences prognosis. Underlying etiology is another significant determinant of outcome (Grady 2005, Postgrad Med J 81, 148). "Acute liver failure" occurs when the liver rapidly loses its ability to function. Acute liver failure is a complex multisystemic illness that evolves quickly after a catastrophic insult to the liver leading to the development of encephalopathy. Whereas, more commonly, liver failure develops slowly over the course of years, in acute liver failure, liver failure develops in a matter of days. The underlying etiology and the pace of progression strongly influence the clinical course. Common causes are, for instance, paracetamol, idiosyncratic drug reactions, hepatitis B and seronegative hepatitis, amatoxins or phallotoxins, both from Amanita species

The term "chronic liver disease" as used herein denotes a disease process of the liver that involves progressive destruction and regeneration of the liver parenchyma leading to fibrosis and cirrhosis. Chronic liver disease causes can be any condition that results in the gradual degradation and renewal of the tissue cells with a body's liver. This process usually results in fibrosis or cirrhosis and can be potentially fatal in cases of chronic liver failure. The classification of the sources of chronic liver diseases fall into five groupings: (i) viral causes such as hepatitis B and C or cytomegalovirus or Epstein Barr virus, (ii) metabolic causes such as Haemochromatosis, non-alcoholic fatty liver disease or Wilson's disease, (iii) autoimmune response causes such as autoimmune chronic hepatitis, primary biliary cirrhosis or primary sclerosing cholangitis, (iv) toxin-related causes such as alcoholic liver disease or nitrofurantoin, amiodarone, or methotrexate, and (v) other miscellaneous causes such as right heart failure. However, the main cause of chronic liver disease is overuse of alcohol, leading to cirrhosis and hepatitis. Therefore, the highest risk group is people who are prone to alcohol abuse. The symptoms associated with chronic liver disease depend on the level of degeneration within the liver.

The beginning stages are usually symptomless and can only be detected by specific medical tests as defined elsewhere herein. Liver diseases that have processed to hepatitis can be recognized by mental confusion, severe jaundice, blood clotting problems, or intestinal bleeding. Those cases that have reached the level of cirrhosis can be noted by the following: nerve problems, male breast growth, Dupuytren's contractions, hair loss, kidney failure, redness of palms, lack of appetite, testicular shrinkage, weakness, weight loss, itching, gallstones, and ascites. Cirrhosis is regarded as a possible end stage of many liver diseases and occurs when healthy liver tissue becomes damaged and is replaced by scar tissue. The replacement process does not happen at once, but takes place over a gradual course of time. The new scarred tissue prevents the regeneration or healing of liver cells. The liver will lose the ability to function as the scarred tissue spreads. Around 10% of all heavy drinkers will eventually reach the stage of cirrhosis. It is thought that cirrhosis is generally reached after ten years of heavy drinking or more. Unfortunately, once a patient has sustained damage to the liver, that damage is not reversible. However, the composition and kit of the invention comprising human adipose stem cells can advantageously be used for the amelioration or treatment of acute or chronic liver diseases.

The term "subject" as used herein includes a mammal such as a rat, pig, cattle, horse, sheep, particularly a human.

Using a model of a chronic toxic liver damage in rats, the inventors have investigated the ability of the diseased hepatic tissue to integrate transplanted adipose stem cells. By repetitive intraperitoneal application of retrorsine and allylalcohol, endogenous hepatocyte proliferation was inhibited and liver necrosis generated. Histologically, hepatic damage due to allylalcohol is periportal and resembles viral liver failure which is the most frequent cause for this organ failure in humans. To simulate a reduction in functional liver tissue and stimulate regeneration, a two-third hepatectomy was performed. Human adipose stem cells were directly injected into one remaining liver lobe. As mentioned elsewhere herein, the direct administration of adipose stem cells turned out to be decisive for a successful implantation and liver regeneration. In fact, the prior art such as Banas et al., loc. cit., teaches the administration of adipose stem cells via the tail vein of mice. Cyclosporine was applied to achieve immunotolerance towards the allogenic stem cells. Every week after surgery, blood samples were drawn to determine multiple liver-correlated blood values. Two, four, six, eight, and twelve weeks after surgery, animals were sacrificed and histological sections were analyzed. The inventors found that human adipose stem cells significantly raised post-operative albumin and total protein levels. Transplanted cells could be found up to eight weeks post surgery in histological sections migrating from the center of the lobe to the periphery. There was no elimination of human adipose stem cells by macrophages. Again, this success is in contrast to the prior art such as Banas et al., loc. cit., who could not observe stem cells in the liver. Rather, these authors observed that stem cells are, for example, in the bile duct which is an indication that the stem cells did either not reach the liver or were removed from the liver. The data of the invention demonstrate that the therapeutic use of human adipose stem cells provides an intriguing alternative to hepatocyte or liver organ transplantation in patients with severe liver failure, particularly in light of the fact that liver cirrhosis is the most important cause of chronic liver failure in humans and in view of the persisting lack of donor organs and the concomitant risks in allotransplantations.

In sum, in contrast to the prior art such as Banas, loc. cit., the present inventors observed long-term survival of stem cells as well as proliferation and thus liver regeneration. Thus, without being bound by theory, the route of administration of adipose stem cells seems to play an important role. This may also be derivable from a clinical study that was stopped. In particular, a clinical trial (identifier NCT01062750 "Liver Regeneration Therapy by Intrahepatic Arterial Administration of Autologous Adipose Tissue Derived Stromal Cells" was suspended. In this trial adipose stem cells were administered to patients via intrahepatic arterial catheterization. However, apparently no success was observed in liver cirrhosis patients. Hence, again the route of administration applied in this study is different from the route chosen by the present inventors and the present inventors observed a success.

Further advantages of the means of the present invention may be briefly summarized as follows: Human adipose tissue as a potential source of adipose stem cells is nearly ubiquitously available. The adipose stem cells are easy to isolate from as described herein. Furthermore, harvesting adipose tissue by, e.g. liposuction generally causes no serious damage to the subject. Adipose stem cells do survive longer in culture and have a higher proliferation activity compared to bone marrow-derived stem cells (BMSC). In addition, BMSC have been shown to be a crucial component in developing liver fibrosis during liver injury, presumably due to interleukin- 10 mediation (Lan et al. 2008, Transpl Int 21, 58192). In contrast, the inventors did not see evidence of enhanced liver fibrosis in rats treated with adipose stem cells. The adipose stem cells can be cultured or cryopreserved. Further, adipose the stem cells can be used in differentiated or non-differentiated or (pre-) differentiated form or can be used in genetically modified form, while the non-differentiated form is preferred. Advantageously, the adipose stem cells as described herein can be used for (i) the regeneration of diseased liver, (ii) the accelerated healing after partial resection of the liver, (iii) an accelerated or increased recovery of the function of the liver in liver insufficiency, (iv) mediating a reduced risk of the occurrence of liver failures, and/or (v) conferring a reduced effect of metabolic disorders. At the same time, negative side effects are minimized by treatment of liver diseases with adipose stem cells in comparison to conventional therapies, such as liver transplantation. For instance, autologous adipose stem cells as defined herein can be used to treat a patient's liver disease, thereby avoiding an immune response against the therapeutic stem cells. In light of the above, adipose stem cells represent an easier, more efficient and safer way than whole organ transplantation to cure patients suffering from liver disease. Without being bound to theory, the functional benefits of adipose stem cells may be because of the functional support of the transplanted or injected cells. Fusion with host hepatocytes is also not excluded. Likewise, the support and activation of endogenous progenitors is possible.

An issue concerning the therapeutic use of the human adipose stem cells or progenitors thereof is the quantity of cells necessary to achieve an optimal effect. The dosage for administration may be variable, may include an initial administration followed by subsequent administrations, and can be ascertained by the skilled artisan armed with the present disclosure. Typically, the administered dose or dosage will provide for a therapeutically effective amount of the cells, i.e., one achieving the desired local or systemic effect and performance. In human studies of autologous mononuclear bone marrow cells, empirical doses ranging from 1 to 4 × 10⁷ cells have been used with encouraging results. However, different scenarios may require optimization of the amount of administered human adipose stem cells or progenitors, thus the quantity of cells to be administered will vary for the subject being treated. In a preferred embodiment, between 10² to 10⁹, or between 10³ to 10⁹, or between 10⁴ to 10⁹, such as between 10⁴ and 10⁸, or between 10⁵ and 10⁷, e.g. about 1 x 10⁵, about 5 x 10⁵, about 1 x 10⁶, about 2 x 10⁶, about 5 x 10⁶, about 1 x 10⁷, or about 2 x 10⁷, about 3 x 10⁷, about 4 x 10⁷, about 5 x 10⁷, about 6 x 10⁷, about 7 x 10⁷, about 8 x 10⁷, about 9 x 10⁷, or about 1 x 10⁸ cells can be administered to a human subject. Even more preferred, the number of adipose stem cells to be administered to the patient is sufficient to repopulate at least 1, 2, 3, 4, 5, 6, 7, 8, 9, 10, 15, 20, or 25% or more such as 30, 35, 40, 45, 50% or even more such as more than 50% of the patient's hepatic mass. However, the precise determination of a therapeutically effective dose may be based on factors individual to each patient, including their size, age, size of tissue damage, and amount of time since the damage occurred, and can be readily ascertained by those skilled in the art from this disclosure and the knowledge in the art.

Preferably, purity of the human adipose stem cells or progenitors thereof or a cell population comprising said cells for administration may be about 50 to about 55 %, about 55 to about 60 %, and about 65 to about 70 %. More preferably, the purity may be about 70 to about 75 %, about 75 to about 80 %, about 80 % to about 85 %; and most preferably, the purity may be about 85 to about 90 %, about 90 % to about 95 %, and about 95 % to about 100 %. Purity of the adipose stem cells can be determined, e.g., according to the cell surface marker profile set forth herein within a cell population. For the determination of purity of the adipose stem cells, for example, FACS analysis can be carried out. Dosages can be readily adjusted by those skilled in the art. For example, lower purity may require an increase in dosage.

The skilled artisan can readily determine the amount of cells and optional additives, vehicles, and/or carrier in compositions of the invention. Typically, any additives (in addition to the active progenitor or adipose stem cell(s) and/or cytokine(s)) may be present in an amount of 0.001 to 50 % (w/w or w/v) solution in phosphate buffered saline, and the active ingredient may be typically present in the order of micrograms to milligrams, such as about 0.0001 to about 5 % (w/w or w/v), preferably about 0.0001 to about 1 %, most preferably about 0.0001 to about 0.05 % or about 0.001 to about 20 %, preferably about 0.01 to about 10 %, and most preferably about 0.05 to about 5 %.

When administering a therapeutic composition of the present invention, it may generally be formulated in a unit dosage injectable or transplantable form (e.g., solution, suspension, dispersion, emulsion). The pharmaceutical formulations suitable for injection include sterile aqueous solutions and dispersions. As used herein, the solutions or dispersions include a pharmaceutically acceptable carrier or diluent in which the adipose stem cells remain viable such as serum or culture medium, e.g., as described herein. The carrier can be a pharmaceutically acceptable solvent or dispersing medium containing, for example, water, saline, phosphate buffered saline, polyol (for example, glycerol, propylene glycol, liquid polyethylene glycol, and the like) and suitable mixtures thereof.

Additionally, various additives which enhance the stability, sterility, and isotonicity of the compositions, including antimicrobial preservatives, antioxidants, chelating agents, and buffers, can be added. Prevention of the action of microorganisms can be ensured by various antibacterial and antifungal agents, for example, parabens, chlorobutanol, phenol, sorbic acid, penicillin, streptomycin, and the like.

In many cases, it will be desirable to include isotonic agents to ensure viability of the cells, for example, sugars, sodium chloride, and the like. The desired isotonicity of the composition of this invention may be accomplished using sodium chloride, or other pharmaceutically acceptable agents such as dextrose, boric acid, sodium tartrate, propylene glycol or other inorganic or organic solutes. Sodium chloride is preferred particularly for buffers containing sodium ions.

To potentially increase cell survival when introducing the progenitor or stem cells or differentiated progeny of interest thereof into a subject in need thereof, may be to incorporate or embed the said cells into a biomaterial, preferably comprising a matrix, comprising, e.g., a biopolymer or synthetic polymer. Examples of suitable biopolymers include, but are not limited to, fibronectin, fibrin, fibrinogen, thrombin, collagen, proteoglycans, chitosane, alginate and hyaluronic acid. An example for a synthetic polymer is polyethylene glycol. This could be constructed with or without included cytokines, growth factors, differentiation factors or nucleic acid expression constructs, etc. Such polymers could be, e.g., in suspension or could form a three-dimensional gel with the cells embedded there within. Such polymers can be preferably biodegradable. Combinations of different polymers are also encompassed by some preferred embodiments of the disclosure.

Prolonged absorption of the injectable pharmaceutical form can be brought about by the use of agents delaying absorption, for example, aluminum monostearate and gelatin. According to the present invention, however, any vehicle, diluent, or additive used would have to be compatible with the progenitor or adipose stem cells.

Sterile injectable solutions can be prepared by incorporating the adipose stem cells utilized in practicing the present invention in the required amount of the appropriate solvent with various amounts of the other ingredients, as desired.

Such compositions may further be in admixture with a suitable carrier, diluent, or excipient such as sterile water, physiological saline, glucose, dextrose, or the like. The compositions can contain auxiliary substances such as wetting or emulsifying agents, pH buffering agents, gelling or viscosity enhancing additives, preservatives, flavoring agents, colors, and the like, depending upon the route of administration and the preparation desired.

Standard texts, such as "Remington's pharmaceutical science", 17th edition, 1985, incorporated herein by reference, may be consulted to prepare suitable preparations, without undue experimentation.

Viscosity of the composition of the invention, if desired, can be maintained at the selected level using a pharmaceutically acceptable thickening agent. Methylcellulose is preferred because it is readily and economically available and is easy to work with. Other suitable thickening agents include, for example, xanthan gum, carboxymethyl cellulose, hydroxypropyl cellulose, carbomer, and the like. The preferred concentration of the thickener will depend upon the agent selected. The point is to use an amount, which will achieve the selected viscosity. Viscous compositions are normally prepared from solutions by the addition of such thickening agents.

The present invention also relates to means for the prevention, amelioration or treatment of acute or chronic liver disease, comprising administration of human adipose stem cells in their non-differentiated form or in a differentiated form, or a progenitor of human adipose stem cells, cell lines thereof or cell populations comprising such, undifferentiated or differentiated human adipose stem cells, optionally genetically modified, to a subject, especially human, in need of such treatment. Such administration is typically in a therapeutically effective amount, i.e., generally an amount which provides a desired local or systemic effect and performance. In another preferred embodiment, the invention relates to a pharmaceutical composition comprising human adipose stem cells in their non-differentiated form or in a differentiated form, or a progenitor of human adipose stem cells, cell lines thereof or cell populations comprising such, undifferentiated or differentiated human adipose stem cells, optionally genetically modified. Preferably, the pharmaceutical composition is for the treatment, amelioration or prevention of acute or chronic liver disease as defined herein. By means of example and not limitation, such cells can be advantageously administered via injection, encompassing also catheter administration, or implantation, e.g. localized injection, systemic injection, intrasplenic injection, injection to a portal vein, injection to the liver pipe, e.g. beneath the liver capsule, parenteral administration, intrauterine injection into an embryo or a fetus, intrahepatic infusion or injection, intraperitoneal infusion or injection, transplantation of biomaterial comprising such cells, or by punction of the liver or the liver parenchyma. The adipose stem cell as defined herein is administered by an administration selected from intrahepatic infusion or injection. The administration may in another preferred embodiment be supported by imaging techniques such as ultrasound, magnetic resonance tomography or computer tomography. The cells may be provided as a cell suspension in any preservation medium, preferably containing human albumin, after isolation procedure or after thawing following cryopreservation.

In one preferred embodiment of the composition of the invention, said acute or chronic liver disease is selected from loss of liver function, ischemia, fibrosis and/or cirrhosis.

In another preferred embodiment of the composition of the invention, the acute or chronic liver disease is selected from the group consisting of: liver ischemia, liver fibrosis, liver cirrhosis, acute liver failure, alcohol liver disease, Alpha-1-antitrypsin deficiency, autoimmune hepatitis, bile duct obstruction, chronic liver failure, chronic hepatitis, cirrhosis, cholestatic liver disease, cystic disease of the liver, enlarged liver, fatty liver, galactosemia, gallstones, Gilbert's syndrome, hemochromatosis, hepatitis A, hepatitis B, hepatitis C, hepatitis D, hepatitis E, liver adenoma, liver cancer, liver hemangioma, liver nodule (focal nodular hyperplasia), neonatal hepatitis, non-alcoholic liver disease, non-alcoholic steatohepatitis, parasitic infection, porphyria, portal vein thrombosis, primary biliary cirrhosis, primary sclerosing cholangitis, Reye's syndrome, sarcoidosis, steatohepatitis, toxic hepatitis, tyrosinemia, type I glycogen storage disease, viral hepatitis, Wilson's disease, medicament-induced liver damage, liver damage induced by a toxin, or any combination thereof. In addition, the composition and kit of the invention are particularly suitable for the amelioration or treatment of acquired liver disorders due to viral infections or liver failure due to loss of liver cells or tissue, such as caused, e.g., by injury or surgery.

I Accordingly, in another preferred embodiment, the human adipose stem cells, progenitors of human adipose stem cells, cell lines thereof or cell populations comprising such, undifferentiated or differentiated human adipose stem cells, optionally genetically modified as detailed herein, for use in therapy and/or use thereof for the manufacture of a medicament for the treatment of acute or chronic liver diseases. Such diseases may include disorders affecting liver tissue. Diseases affecting the hepatocytic viability and/or function are specifically contemplated, and may represent, e.g., inborn errors, acquired liver diseases, therapy-induced liver damage, the effect of a disease condition, the effect of trauma, toxic effects, or viral infections. Liver diseases listed in the present specification are specifically contemplated. Administration of the human adipose stem cells according to the invention can lead to liver tissue reconstitution and/or regeneration in the subject and/or alleviating the symptoms of a liver disease such as one described herein. More specifically, by using the composition or kit of the invention comprising the adipose stem cells described herein, regeneration of the liver, accelerated healing after partial resection of the liver, accelerated or increased recovery of the liver function in liver insufficiency, or a reduced risk of occurrence of hepatic dysfunctions or hepatic impairment or a reduced effect of metabolic diseases can be achieved. The cells are administered in a manner that permits them to graft and migrate to the intended tissue site and reconstitute or regenerate the functionally deficient area in the liver.

In one preferred embodiment of the composition of the invention, the adipose stem cell is isolated from human adipose tissue or any other suitable source comprising adipose stem cells,

The isolation of adipose stem cells is well known in the art and further specified elsewhere herein. For example, adipose stem cells can be isolated from subcutaneous or peritoneal adipose tissue, or other suitable human tissue.

In a further preferred embodiment of the composition of the invention, the adipose stem cell is an autologous, heterologous or xenologous adipose stem cell.

The term "autologous" adipose stem cell as used herein means that the adipose stem cell is derived from the same individual. In this preferred embodiment, the present invention advantageously contemplates using a patient's own tissue, such as adipose tissue, or other suitable tissue to isolate the human adipose stem cells or progenitors of human adipose stem cells. Such cells would be autologous to the patient and could be readily administered to the patient, without eliciting an immune response and/or without running the risk of transmitting a causative agent of a disease such as HIV or HCV. Moreover, if the patient carried a genetic defect underlying a particular pathological condition, such defect could be averted by genetically manipulating the obtained adipose stem cells. In another preferred embodiment, the human adipose stem cells or progenitors of human adipose stem cells may be isolated from tissue which is not the patient's own. In this case, the adipose stem cells are heterologous adipose stem cells.

The term "heterologous" as used herein denotes that the adipose stem cell is derived from another individual. Where administration of such cells to a patient is contemplated, it may be preferable that tissue subjected to a method to obtain the adipose stem cell or progenitor is selected such as to maximize, at least with achievable limits, the tissue compatibility between the patient and the administered cells, thereby reducing the chance of rejection of the administered cells by the patient's immune system.. If the cells are derived from a heterologous, i.e. non-autologous source, concomitant immunosuppression may be typically administered, e.g., using immunosuppressive agents, such as cyclosporine or FK506.

The term "xenologous" as used herein denotes that the adipose stem cell is derived from a species other than the individual to whom the adipose stem cell is intended to be administered. For example, an adipose stem cell may be isolated from swine and administered to a human.

In a further preferred embodiment of the composition of the invention, the adipose stem cell is a mesenchymal stem cell.

The term "mesenchymal stem cell" as used herein means multipotent stem cells capable of differentiating into mesenchymal cells, such as adipocytes, osteoblasts and chondrocytes, but also myocytes, neurons, endothelial cells, astrocytes and epithelial cells. Although first reported in the normal adult bone marrow, mesenchymal stem cells can also be obtained from other sources, such as umbilical cord blood, from the dermis or dental pulp, bone marrow and adipose tissue. The definition for mesenchymal stem cells as used herein requires minimal criteria including, but not limited to, e.g., plastic adherence, multiple differentiation potential, expression of cell surface markers such as CD105 and lack of expression of CD14, CD34 and CD45 (Pittenger et al. 1999, Science 284, 143).

In a preferred embodiment of the composition of the invention, the mesenchymal stem cell is positive for the cell surface markers CD13, CD29, CD49a, CD63, CD73, CD90, CD105 and/or CD166 and/or negative for the cell surface markers CD31, CD34, CD44, CD45 and/or CD106.

As set forth in more detail elsewhere herein, mesenchymal stem cells are multipotent stem cells that can differentiate into a variety of cell types. Mesenchymal stem cells can be readily isolated and/or do not show a strong tendency to become degenerated. In this embodiment, the mesenchymal stem cell is further characterized by a specific cell surface marker profile. The term "positive for" as used herein with respect to cell surface markers means that, in a cell population, more than 20%, 30%, 40%, 50%, 60%, 70%, 80%, 90%, 95%, 99% or all cells express said marker. The term "negative for" as used herein with respect to cell surface markers means that, in a cell population, less than 20%, 10%, 9%, 8%, 7%, 6%, 5%, 4%, 3%, 2%, 1% or none of the cells express said marker. Expression of cell surface markers can be determined, for example, by flow cytometry, immunohistochemistry, ELISA, RT-PCR, Northern or Western blot, for (a) specific cell surface marker(s) using conventional methods described in the art.

In another preferred embodiment of the composition of the invention, the adipose stem cell is an undifferentiated mesenchymal stem cell, a differentiated mesenchymal stem cell or a genetically modified mesenchymal stem cell.

According to this embodiment, the adipose stem cell can be an undifferentiated, i.e. non-differentiated mesenchymal stem cell (MSC), a (pre-)differentiated mesenchymal stem cell or a genetically modified mesenchymal stem cell. The mesenchymal cell can be administered in the composition or kit of the invention in an undifferentiated status, for example, directly after its isolation from adipose tissue. Undifferentiated MSC have been found to be less receptive to oxidative stress than MSC-derived hepatocytes and, therefore, more likely to survive the initial hypoxic phase after transplantation (Kuo et al. 2008, Gastroenterology 134, 2111). Additionally, using undifferentiated adipose stem cells is less costly, implements less handling steps and culture components being less risky for contaminations and means less time wasted in cell culture with having the possibility to quickly start urgently needed therapy.

In another embodiment, the isolated mesenchymal stem cell can be differentiated or pre-differentiated *in vitro*, before being administered to the patient having a liver disease. By using the (pre-)differentiated adipose stem cells, the integration of said stem cells into the diseased liver and/or replacement of non-functional liver cells may be more efficient. For instance, the adipose stem cells can undergo adipogenic, osteogenic or hepatogenic differentiation, by choosing appropriate cell culture conditions, as exemplified in the following examples; see also Aurich et al., loc. cit

In addition, the non-differentiated or (pre-)differentiated adipose stem cells can be genetically modified, if necessary, in order to compensate for, e.g., a genetic defect in the liver cells of a patient; see, for example, EP 2281875. Gene transfer methods for human patients are described, e.g., in Alaee et al. (2011) Genetic vaccines and therapy;9:4; Sellneret al. (2011) Leukemia & lymphoma;52(3):483-90; Grez (2011) Mol Ther;19(1):28-35.

The terms "differentiation", "differentiating" or derivatives thereof as used herein denote the process by which an unspecialized or a relatively less specialized cell becomes relatively more specialized. In the context of cell ontogeny, the adjective "differentiated" is a relative term. Hence, a "differentiated cell" is a cell that has progressed further down a certain developmental pathway than the cell it is being compared with. A differentiated cell may, for example, be a terminally differentiated cell, i.e., a fully specialized cell that takes up specialized functions in various tissues and organs of an organism, and which may but need not be post-mitotic. In another example, a differentiated cell may also be a progenitor cell within a differentiation lineage, which can further proliferate and/or differentiate.

Similarly, a cell is "relatively more specialized" if it has progressed further down a certain developmental pathway than the cell it is being compared with, wherein the latter is therefore considered "unspecialized" or "relatively less specialized". A relatively more specialized cell may differ from the unspecialized or relatively less specialized cell in one or more demonstrable phenotypic characteristics, such as, for example, the presence, absence or level of expression of particular cellular components or products, e.g., RNA, proteins, specific cellular markers or other substances, activity of certain biochemical pathways, morphological appearance, proliferation capacity and/or kinetics, differentiation potential and/or response to differentiation signals, etc., wherein such characteristics signify the progression of the relatively more specialized cell further along the said developmental pathway.

Non-limiting examples of differentiation may include, e.g., the change of a pluripotent stem cell into a given type of multipotent progenitor or stem cell, the change of a multipotent progenitor or stem cell into a given type of unipotent progenitor or stem cell, or the change of a unipotent progenitor or stem cell to more specialized cell types or to terminally specialized cells within a given cell lineage. Differentiation of an unspecialized or less specialized cell to a more specialized cell may proceed through appearance of cells with an intermediate degree of specialization. For example, the isolated mesenchymal stem cell may be differentiated *in vitro* to adipocytes, hepatocytes, or osteoblasts by using appropriate growth and differentiation factors and cell culture components ("Adipose-Derived Stem Cells - Methods and Protocols" Jeffrey M. Gimble and Bruce A Bunnell (Editors), Methods in Molecular Biology 702, Springer Protocols 2011; Lee et al. (2004) Hepatology 40(6):1275-84.). For instance, adipogenic differentiation of the adipose stem cells can be induced by the alternated use of basal medium such as 5% FCS/DMEM supplemented with IDI-mix, i.e. 500 µM 3-isobutyl-1-methylxanthine, 1 µM dexamethasone, 1 µM indomethacin, for 2 days followed by basal medium plus 10 µg/ml insulin for 1 day, as described in the following examples. The induction cycle is repeated 3 times. Adipogenic differentiation can be analyzed using oil red quantification. Osteogenic induction can be initiated by changing the medium to DMEM containing 5 % FCS, supplemented with 50 µM L-ascorbate-2-phosphate, 0.1 µM dexamethasone and 10 mM β-glycerophosphate disodium. Calcium deposition can be demonstrated histochemically by alizarin red stain as described in the attached examples.

The induction of differentiation into hepatocytes or hepatocyte-like cells can be carried out by cultivating adipose stem cells for about two weeks on collagen type I coated dishes and treatment with 20ng/ml Activin A and 20ng/ml fibroblast growth factor 4 (FGF4) for three days, followed by treatment with 150ng/ml hepatocyte growth factor (HGF), 100ng/ml FGF1, 30ng/ml oncostatin M, 2x10⁻⁵ mol/L dexamethasone, 1 x insulin-transferrin-selenium (ITS), 0.1% dimethyl sulfoxide (DMSO) and 0.05mmol/L nicotinamide for ten days, and maintenance in hepatocyte culture medium alone or, optionally, with 10⁻⁸ mol/L dexamethasone and 0.05 mmol/L nicotinamide, as detailed, e.g., in Aurich et al. 2009, Gut 58, 570; Banas et al. 2008, J Gastroenterology and Hepatology 24, 70.

Accordingly, in a further preferred embodiment of the composition of the invention, the differentiated mesenchymal stem cell is an adipogenically, osteogenically or hepatogenically differentiated mesenchymal stem cell.

Differentiation of adipose stem cells into adipogenically or osteogenically differentiated cells can be induced by defined cell culture conditions, as set forth above and demonstrated in the following examples. Further encompassed by the composition of the invention is the differentiation of adipose stem cells into hepatogenically differentiated cell, i.e. hepatocytes or hepatocyte-like cells, as described, e.g., by Aurich et al. (Gut 2009, 58, 570). The adipose stem cells can in one embodiment be differentiated *in vitro* before being administered to a subject having an acute or chronic liver disease. Preferably, the adipose stem cell as referred to herein is differentiated into an adipogenically differentiated cell, such as an adipocyte, or into an osteogenically differentiated cell, such as an osteoblast, or into a hepatogenically differentiated cell, such as an hepatocyte, as further detailed herein below and in Aurich et al., loc. cit. It is preferred that said adipose stem cell differentiated into an adipogenically differentiated cell is characterized by accumulation of intracellular triacylglycerines and/or lipid droplets and/or expression of at least one gene specific for adipocytes, such as - without limitation - lipoprotein lipase or peroxisome proliferator-activated receptor γ2 (PRARγ2) or GAPDH. It is also preferred that said adipose stem cell differentiated into an osteogenically differentiated cell is characterized by extracellular calcium phosphate depositions and/or the expression of at least one gene specific for osteoblasts, such as - without limitation - osteonectin or osteocalcin. Preferably, said adipose stem cell differentiated into a hepatocyte exhibits at least one hepatocyte-specific marker, such as - without limitation, albumin, CD26, CD29, HepPar1, carbamoylphosphate synthetase, tryptophan 2,3-dioxygenase, P450 type 3A4 (CYP3A4), FOXA2 or asialoglycoprotein receptor (AGPR)., and/or at least one hepatocyte-specific function, such as - without limitation, e.g., urea formation, purine metabolism, cytochrome P450 enzyme activity, protein synthesis and storage, transformation of carbohydrates, synthesis of cholesterol, bile salts and phospholipids, detoxification, modification and excretion of exogenous and endogenous substances, formation and secretion of bile, uptake of low-density lipoprotein or synthesis and storage of glycogen. Said hepatocyte-differentiated adipose stem cells are preferably negative for hepatocyte progenitor cell markers such as CK7 or CX43. Other hepatocyte-specific markers and functions are well known in the art (Sato et al. (2005) Blood 106(2):756-63; Lee et al. (2004) Hepatology 40(6):1275-84.

In another embodiment of the composition of the disclosure the composition further comprises a matrix in which the cells are embedded.

According to this embodiment, the adipose stem cells as defined herein are embedded into a matrix, comprising, e.g., a biopolymer or synthetic polymer, in order to potentially increase cell survival when introducing the stem cells into a subject in need thereof, for example, by providing a three-dimensional structure to the adipose stem cells. Suitable biopolymers include, without limitation, fibronectin, fibrin, fibrinogen, thrombin, collagen, and proteoglycans. As a synthetic polymer, for instance, polyethylene glycol can be utilized. The matrix can in some embodiments comprise, in addition to the adipose stem cells, cytokines, growth factors, differentiation factors or nucleic acid expression constructs and components of the extracellular matrix, etc. Such polymers can be, e.g., in suspension or they can form a three-dimensional gel with the cells embedded there within. Preferably, such polymers can be biodegradable. Combinations of different polymers are also encompassed by some preferred embodiments of the invention. In another embodiment, the matrix comprises a hydrogel.

Preferably, the matrix comprises polyethylene glycol, collagen, fibrin, or a combination thereof.

In still another preferred embodiment of the composition of the invention, the composition further comprises a biomaterial, on which the cells are coated.

Preferably, the biomaterial is fibronectin, fibrin, fibrinogen, thrombin, collagen, proteoglycans, chitosane, alginate and hyaluronic acid alginate, chitosane, hyaluronic acid, polyethylene glycol or any other biomaterial as described herein.

In still another embodiment of the composition of the invention, the composition further comprises serum or other blood components, such as plasma.

Plasma or serum often contain cellular factors and components that are necessary for cell viability and expansion. Plasma is usually obtained from a sample of whole blood, which is provided or contacted with an anticoagulant, such as heparin, citrate or EDTA, upon or shortly after drawing the blood sample, to prevent clotting. Subsequently, cellular components of the blood sample are separated from the liquid component (plasma) by centrifugation. Serum can be obtained from plasma by removing the anticoagulant and fibrin.

Preferably, the serum is human serum or human serum from platelet-poor plasma.

In still another preferred embodiment of the composition of the invention, the adipose stem cell is (to be) administered (i) directly after isolation, (ii) after cultivation and/or proliferation in cell culture or (iii) after cryopreservation.

According to this embodiment, the adipose stem cell can be used for administration to the patient having a liver disease directly after isolation. The stem cells can also be maintained in cell culture and/or propagated in conditions that allow for growth and doubling of the said cells without differentiation. Such conditions may be, e.g., the ones used for obtaining the stem cells. A skilled person capable of assessing the presence or absence of cell differentiation may readily establish further conditions. This can increase the number of the stem cells available for further use thereof. The present inventors have realized that the primary adipose stem cells or any subsequent passage could be cryopreserved for further use, as generally known in the art for mammalian cells. The present inventors have found that the isolated adipose stem cells substantially retain their proliferation capacity after freezing and thawing. The said cells may be stored as a frozen concentrated cell suspension, thawed, as generally done in the art, and re-plated in the same conditions as described, for instance in the following examples. In another embodiment, the isolated adipose stem cells can be differentiated into more specialized cell types, by using appropriate cell culture conditions, as described elsewhere herein.

In a further preferred embodiment of the composition of the invention, the adipose stem cell is (to be) administered by intrahepatic infusion/injection, supported by imaging techniques such as ultrasound, magnetic resonance tomography or computer tomography.

In still further preferred embodiment of the composition of the invention, the total protein level and/or the albumin level in the serum is increased, in comparison to a non-treated subject.

The total protein level and/or the albumin level in the serum can be determined by methods described in the art, e.g. from blood or serum.

In another embodiment of the composition of the invention, the iron level in the serum is decreased, in comparison to a non-treated subject.

The iron level in the serum can be determined by methods described in the art, e.g. from blood or serum.

Liver function tests are groups of clinical biochemistry laboratory blood assays designed to give information about the state of a patient's liver. The parameters measured include albumin, total protein, iron, cholin esterase, lactate dehydrogenase, glutamic oxaloacetic transaminase, glutamic pyruvic transaminase, alkaline phosphatase, billirubin (direct and indirect) and others. Liver transaminases (aspartate aminotransferase (AST) and alanine aminotransferase (ALT including GPT and AST) are not liver function tests but are biomarkers of liver injury in a patient with some degree of intact liver function. Most liver diseases cause only mild symptoms initially, but it is vital that these diseases be detected early. This testing is performed on a patient's serum or plasma sample obtained by phlebotomy. Some tests are associated with functionality (e.g., albumin); some with cellular integrity (e.g., transaminases) and some with conditions linked to the biliary tract (gamma-glutamyl transferase and alkaline phosphatase). As known to those skilled in the art, several biochemical tests are useful in the evaluation and management of patients with hepatic dysfunction. These tests can be used to (1) detect the presence of liver disease, (2) distinguish among different types of liver disorders, (3) gauge the extent of known liver damage, and (4) follow the response to treatment.

Total protein measurements can be used to screen for and help diagnose liver disease. Low total protein levels can suggest a liver disorder. Higher total protein level in the serum of a patient having a liver disease treated with adipose stem cells is indicative of restoration of the liver function, in comparison to the total protein level in the serum of a non-treated patient having the same liver disease.

Albumin is a protein made specifically by the liver, and can be measured cheaply and easily. It is the main constituent of total protein; the remaining fraction is called globulin, including the immunoglobulins. Albumin levels are decreased in chronic liver disease, such as cirrhosis. Higher albumin level in the serum of a patient having a liver disease treated with adipose stem cells indicates restoration of the liver function, in comparison to the albumin level in the serum of a non-treated patient having the same liver disease.

By using an animal model, the inventors could show in the following examples that the injection of adipose stem cells into the damaged liver after 2/3 hepatectomy leads to a significantly higher and earlier restoration of liver function compared with the non cell treated control group. This could be demonstrated by higher albumin and total protein levels.

Iron levels in serum are elevated in liver diseases and have been used as marker for liver damage in the past. Lower iron levels in an adipose stem cell-treated subject having a liver disease can be interpreted as faster regeneration of the liver, in comparison to the iron level in the serum of a non-treated patient having the same liver disease.

The lower iron levels in cell treated animals 3 weeks post surgery can be interpreted as faster regeneration due to the injected adipose stem cells, as can be derived from the attached examples.

Alanine transaminase or Alanine aminotransferase (ALT), also called serum glutamic pyruvate transaminase (SGPT), is an enzyme present in hepatocytes and used as a marker for liver injury. When a cell is damaged, it leaks this enzyme into the blood, where it is measured. ALT rises dramatically in acute liver damage, such as viral hepatitis or paracetamol overdose. Elevations are often measured in multiples of the upper limit of normal (ULN).

Aspartate transaminase or Aspartate aminotransferase (AST), also called serum glutamic oxaloacetic transaminase (SGOT), is similar to ALT in that it is another enzyme associated with liver parenchymal cells. It is used as a marker for liver injury since it is raised in acute liver damage. Yet, said enzyme is also present in red blood cells, and cardiac and skeletal muscle and is therefore not specific to the liver. The ratio of AST to ALT is sometimes used in differentiating between causes of liver damage.

It is envisaged that ALT, AST, lactate dehydrogenase and/or ammonia are decreased after administration of adipose stem cells to the human patient having a liver disease as defined herein.

Alkaline phosphatase (ALP) is an enzyme in the cells lining the biliary ducts of the liver. ALP levels in plasma will rise with large bile duct obstruction, intrahepatic cholestasis or infiltrative diseases of the liver. However, ALP is also present in bone and placental tissue.

Bilirubin is a breakdown product of haem. The liver is responsible for clearing the blood of bilirubin. Bilirubin is taken up into hepatocytes, conjugated, and secreted into the bile, which is excreted into the intestine. Increased total bilirubin causes jaundice, and can signal hepatic problems which are reflected as deficiencies in bilirubin metabolism, e.g., reduced hepatocyte uptake, impaired conjugation of bilirubin, and reduced hepatocyte secretion of bilirubin. Examples are cirrhosis and viral hepatitis.

Although reasonably specific to the liver and a more sensitive marker for cholestatic damage than ALP, gamma glutamyl transpeptidase (GGT) may be elevated with even minor, sub-clinical levels of liver dysfunction. For instance, GGT is raised in chronic alcohol toxicity. It can also be helpful in identifying the cause of an isolated elevation in ALP.

Liver function tests analyzing the above mentioned parameters are described, e.g., in Manizate F, Hiotis SP, Labow D, Roayaie S, Schwartz M. Liver functional reserve estimation: state of the art and relevance for local treatments: the Western perspective. Journal of hepato-biliary-pancreatic sciences; 17(4):385-8; Martinez SM, Crespo G, Navasa M, Forns X. Noninvasive assessment of liver fibrosis. Hepatology (Baltimore, Md;53(1):325-35; Rodriguez et al. (2010) Nutr Hosp;25(5):712-7.

In still another embodiment of the composition of the invention, the stem cells can be detected for at least 4, 6, or preferably 8 weeks, more preferably longer than 8 weeksafter infusion or transplantation.

As evidenced by the following examples, the injected adipose stem cells migrated from an initially central location within the lobe to its periphery and could be found up to 8 weeks after injection.

The disclosure also relates to an implantable matrix comprising human adipose stem cells for the prevention, amelioration or treatment of acute or chronic liver disease.

The definitions and advantages set forth herein for the composition and kit of the disclosure apply *mutatis mutandis* to the implantable matrix. The matrix can contain polyethylene glycol, collagen, fibrin, or a combination thereof, optionally with a physiologically acceptable buffer.

The disclosure further pertains to a composition in a physiologically compatible buffer, comprising: human adipose stem cells and at least one of (i) a semi solid substance or (ii) a biodegradable substance or (iii) human serum, preferably serum from platelet poor plasma, for the prevention, amelioration or treatment of acute or chronic liver disease.

The semi solid substance or biodegradable substance can be hyaluronic acid, collagen, thrombin, elastin, chondroitin sulfate, albumin or a mixture thereof.

Finally, the disclosure pertains to a kit comprising the composition or the implantable matrix of the invention and an instruction sheet, for the prevention, amelioration or treatment of acute or chronic liver disease. The kit may comprise human adipose stem cells being either autologous or heterologous in relation to the patient described herein as well as means for isolating adipose stem cells and/or means for administering adipose stem cells as described herein, most preferably via intrahepatic infusion or injection. Accordingly, said kit may thus contain an instruction sheet describing the method as how to isolate and/or culture and/or administer the adipose stem cells as described herein.

The instruction sheet contains dosage and administration information on the adipose stem cells, optionally with cell culture conditions for proliferation and/or differentiation of the adipose stem cells. As regards the implantable matrix, it contains information about the implantation of the implantable matrix.

### FIGURES

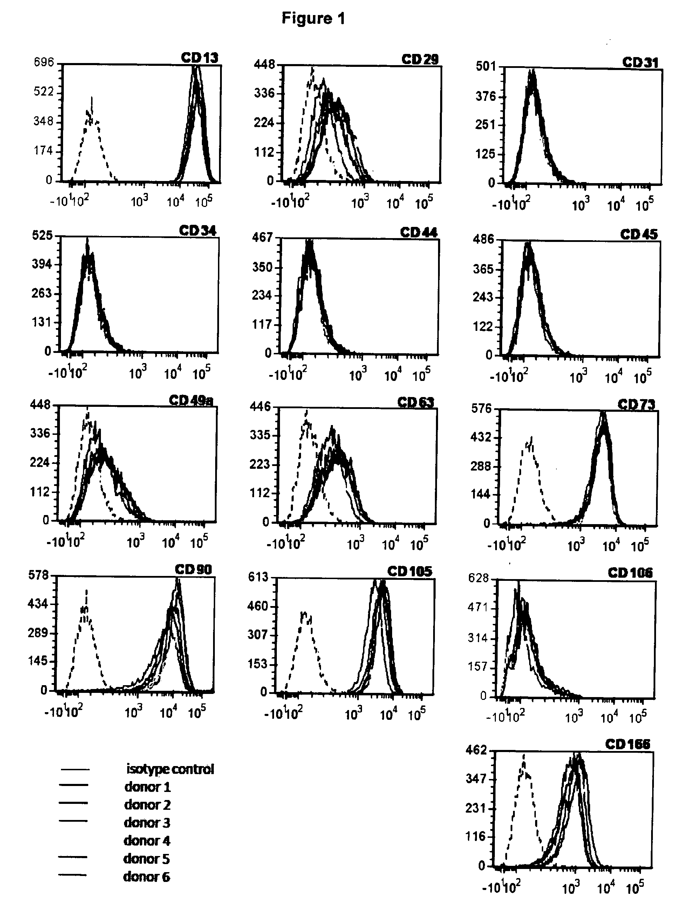

**Figure 1** shows FACS results of human adipose stem cells (hADSC) using the indicated cell surface markers. The stem cells were positive for CD13, CD29, CD49a, CD63, CD73, CD90, CD105 and/or CD166 and/or negative for CD31, CD34, CD44, CD45 and/or CD106.. The cells, therefore, meet the minimal consensus criteria for mesenchymal stem cells.

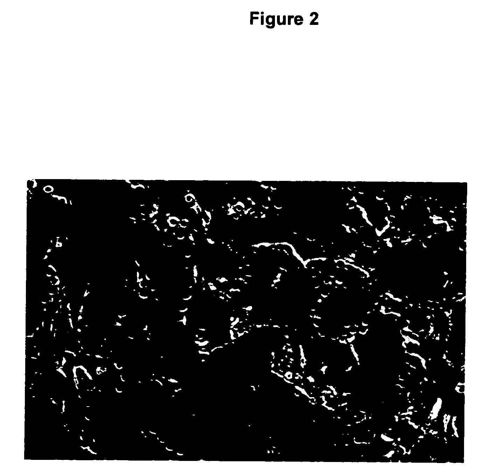

**Figure 2** shows Oil Red staining for accumulation of intracellular triacylglycerines in adipogenically differentiated human adipose stem cells. Adipogenically induced stem cells showed a statistically significant higher oil red concentration than not induced controls in all donors.
**Figure 3** shows Alizarin Red staining for extracellular calcium deposition in osteogenically differentiated human adipose stem cells (hADSC). Osteogenically differentiated hADSC showed high extracellular calcium deposition, analyzed with alizarin red stain. Non-induced cells did not show extracellular calcium deposition.

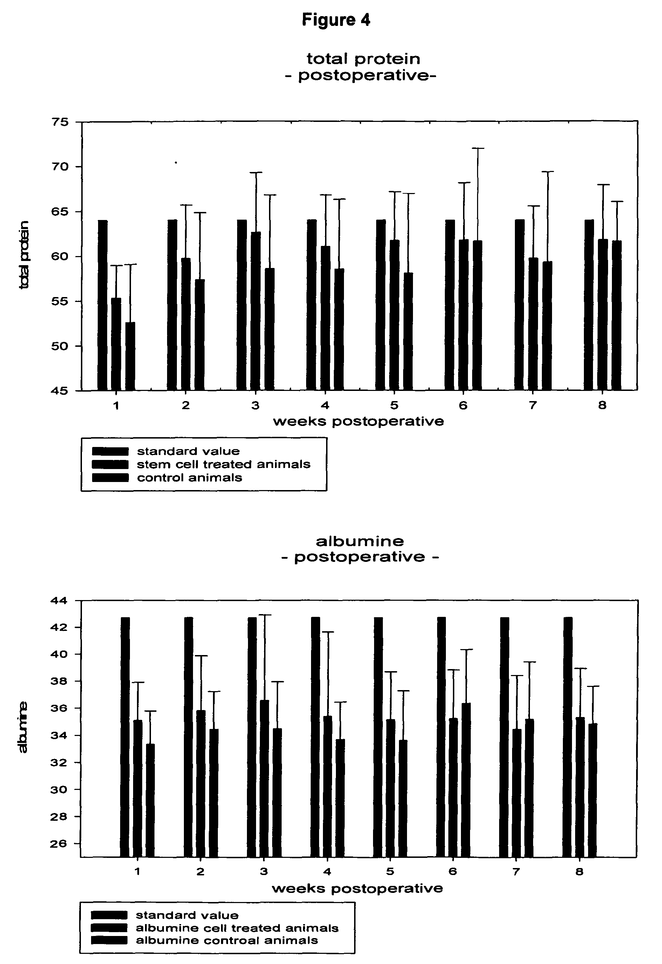

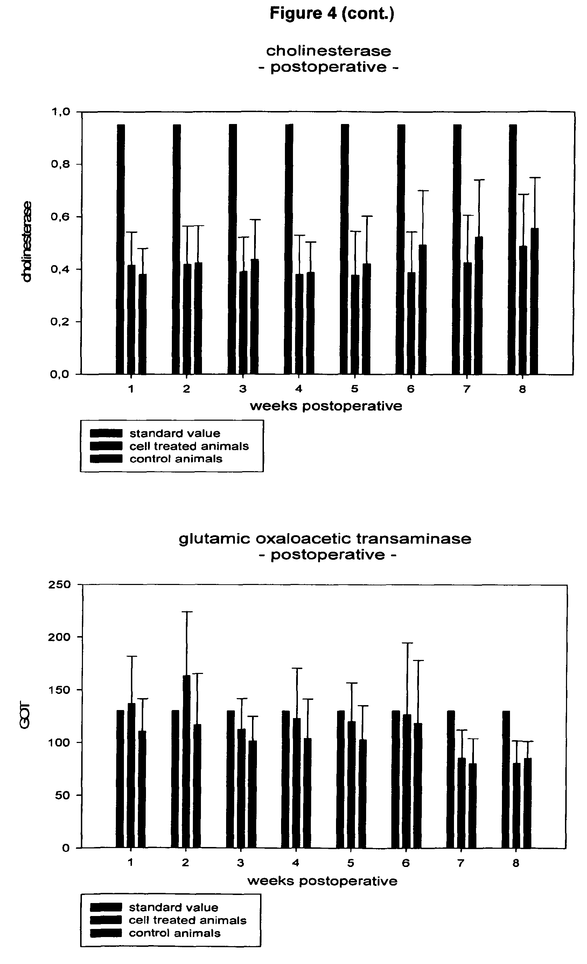

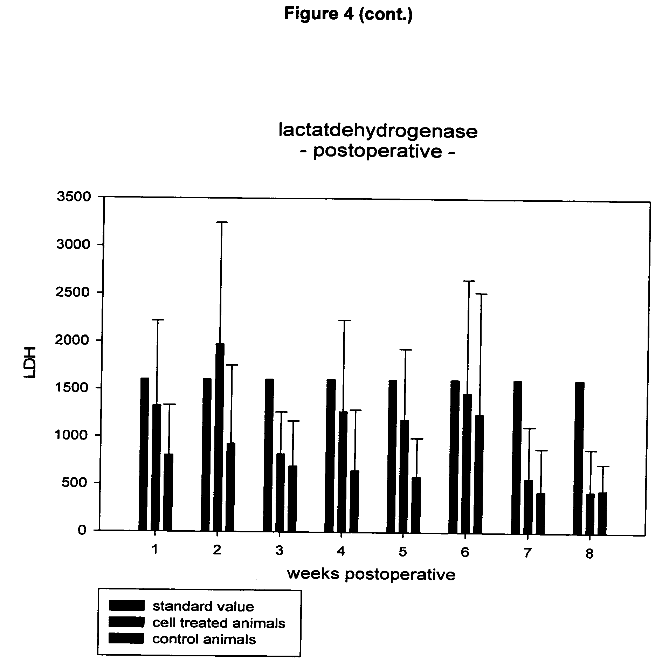

**Figure 4** shows serum levels of albumin, total protein, CHE (cholinesterase), GOT (glutamic oxaloacetic transaminase) and LDH (lactate dehydrogenase). Black bars show normal standard values, red bars values of cell treated animals and green bars values of non cell treated control animals. Data were expressed as mean ± SE. Results were analyzed by the Student's r test and Mann Whitney Rank Sum test when normality test failed. P < 0.05 was considered statistically significant.

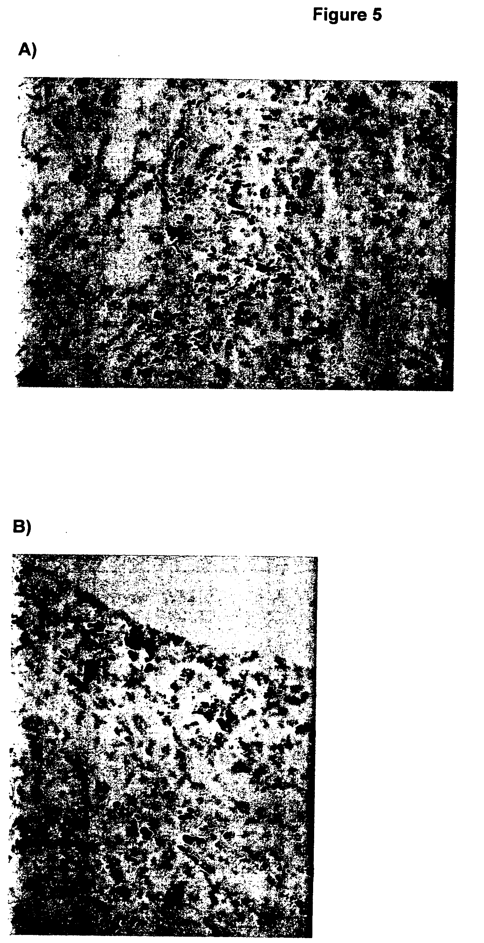

**Figure 5** shows Berliner Blau stain of SPIO (superparamagnetic iron oxide particle)-labeled human adipose stem cells in rat liver lobe sections. a) 2 weeks after injection and b) 4 weeks after injection.

### EXAMPLES

The following examples are put forth so as to provide those of ordinary skill in the art with a complete disclosure and description of how to make and use the embodiments, and are not intended to limit the scope of what the inventors regard as their invention nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, and temperature is in degrees Celsius. Standard abbreviations are used.

### Example 1: Cells

### Isolation of adipose tissue derived mesenchymal stem cells

After informed consent mesenchymal stroma cells were isolated from freshly excised subcutaneous adipose tissue from six adults (females in the range 34-59, with a median age of 39,5 years) undergoing elective plastic surgery using a procedure modified from Hauner et al. (1989). Briefly, after removing fibrous tissue, the adipose tissue was washed two times in 1 % BSA/PBS, minced and digested enzymatically by collagenase, (collagenase CLS; 220 U/mg, Biochrom AG, Berlin, Germany, 1.5 mg/ml, in 1 % BSA/Krebs-Ringer-solution) for 45 minutes under constant shaking at 37 °C. Mature adipocytes and connective tissue were separated by centrifugation (700 x g, 7 min., RT). The sedimented cells were resuspended, passed through a 100 µm mesh filter (Neolab, Heidelberg, Germany) and washed twice with 1 % BSA/PBS. After erythrocyte lysis (3 minutes, 155 mM ammoniumchloride, 10 mM potassium bicarbonate; 0,1 mM EDTA), cells were washed again two times and plated at a density of 2 x 10⁴ cells/cm² in expansion medium. After 12 hours, medium was changed to remove non adhered cells. Expansion medium was replaced every second day.

Cells were cultured in expansion medium (60 % DMEM (Invitrogen, Karlsruhe, Germany)), 40 % MCDB-201 (Sigma), 1 x insulin transferring selenium (Becton Dickinson, Heidelberg, Germany), 10⁻⁸ M dexamethasone, 0,1 mM ascorbic acid-2-phosphate, 2 % fetal calf serum (Biochrom, Berlin, Germany), 100 U/ml penicillin (Biochrom), 0,1 mg/ml streptomycin (Biochrom), 10 ng/ml rhEGF (Miltenyi, Bergisch Gladbach, Germany) and 10 ng/ml rhPDGF-BB (CellSystems, St. Katharinen, Germany). Medium was exchanged every second day. Once the cells reached 70 % confluence, they were detached with 0.25 % trypsin-EDTA (Biochrom, Berlin, Germany) and replated with 3,5 x 10³ cells per cm². Cultures were incubated at 37 °C with 5 % CO₂.

### Example 2: Adipogenic and osteogenic differentiation

### Adipogenic differentiation

Cells were seeded in expansion medium at a density of 24.000 cells/cm². After reaching 90 % confluence, adipogenesis was induced by the alternated use of basal medium (5 % FCS/DMEM) supplemented with IDI-mix (500 µM 3-isobutyl-1-methylxanthine; 1 µM dexamethasone; 1 µM indomethacin) for 2 days followed by basal medium plus 10 µg/ml insulin for 1 day. The induction cycle was repeated 3 times. Adipogenic differentiation was analyzed using oil red quantification as described previously [Ramirez-Zacarias et al. 1992, Histochemistry 97, 493].

### Osteogenic differentiation

After seeding with a density of 24.000 cells/cm², cells were grown in expansion medium to 90 % confluence. Osteogenic induction was initiated by changing the medium to DMEM containing 5 % FCS, supplemented with 50 µM L-ascorbate-2-phosphate, 0.1 µM dexamethasone and 10 mM β-glycerophosphate disodium. Calcium deposition was demonstrated histochemically by alizarin red stain as described previously (Landoff (1948) Acta Orthop Scand. 1948;17(3-4):270-302; Wise et al. (1979) J. Biol. Chem. 54(2):273-5. A typical protocol for the quantification of calcium from osteogenically differentiated MSC is as follows: Cells were analysed 14, 21, 35, and 42 days after induction of differentiation. Cell layers of osteogenically differentiated MSC were washed twice with PBS w/o Mg2+/Ca2+. Extracellular deposited Ca2+ was extracted with 0.6 M HCl by incubating the cells at room temperature on a plate rocker for 2 hours. Extracts were centrifuged for 5 minutes at 15,700 x g and Ca2+-content in the supernatants was quantified by the O-cresolphthalein complex method (Fluitest Ca-CPC, Biocon, Vöhl-Marienhagen, Germany). Subsequently extracted cells were washed three times with PBS w/o Mg2+/Ca2+, scraped with a rubber policeman into 0.1 M NaOH / 1% SDS and sonified as described above. After centrifugation at 4 oC and 8,000 x g for 5 minutes the protein content of the supernatants was determined with the BCA-kit (Pierce, Rockford, USA).

### A typical protocol for alizarin staining is as follows: Monolayers of mineralized MSC were washed twice with excess PBS and fixed with pre-chilled 70% Ethanol for 1 hour at -20 ° C. After a short washing step with H₂O the cell layer was incubated with 40 mM Alizarin red (pH 4.2) for 1 minute at room temperature. After aspiration of unincorporated dye cells were washed twice more with excess H₂O and once with PBS before microscopic analysis

### Example 3: Flow cytometry

Human adipose stem cells (hADSC) expanded to passage four were examined for surface marker expression using flow cytometry. The following monoclonal antibodies (MAbs) conjugated to fluorochromes were used: anti-CD13-APC, anti-CD29-PE, anti-CD31-FITC, anti-CD34-FITC, anti-CD44-APC, anti-CD45-FITC, anti-CD49a-PE, anti-CD63-FITC, anti-CD73-PE, anti-CD90-APC, anti-CD105-FITC, anti-CD166-PE (all from Becton Dickinson). Isotype antibodies were included for all fluorochromes.

Cells were detached with 0.25 % trypsin-EDTA, incubated with directly conjugated MAbs in FACS buffer (1 % FCS, 0.1 % NaN₃, in PBS) for 30 min on ice, washed twice with FACS buffer and fixed with 1 % paraformaldehyde/PBS. Cells were analyzed using a FACSCanto flow cytometry system (Becton Dickinson). Data acquisition and analysis was performed with Diva software (Becton Dickinson).

### Example 4: SPIO labeling

Cells were labeled with superparamagnetic iron particles for *in vivo* tracing. This did not interfere with cell proliferation or differentiation.

### Example 5: Animals

All studies were conducted under protocols approved by the Animal Care Use Committee of the Ruprecht Karls University Heidelberg, Germany, and were in accordance with National Institutes of Health guidelines.

Female Sprague Dawley rats weighing 140-200 g were purchased from Charles River Laboratories. They were maintained on an automatic 12 h light/dark cycle and were fed standard rat chow and water *ad libitum.*

### Application of toxic agents

After 1 week of acclimatization, all rats received two intraperitoneal injections of retrorsine, separated by an interval of 13 days, each of 30 mg/kg body weight. Retrorsine was dissolved in HCl (pH 2.5) followed by neutralization by NaOH 0.1 N, as described (Lan et al. 2008, Transpl Int 21, 58192; Kuo et al. 2008, Gastroenterology 134, 2111).

30 days after the second injection, animals received 10 repeated injections of allylalcohol every third day, each of 0,31-0,372 mmol/kg body weight.. 3 days after the last injection of allylalcohol, all animals underwent a two-third partial hepatectomy; see operative procedure.

### Operative procedure

3 days after the last injection of allylalcohol, all animals underwent a two-third partial hepatectomy (see, for example, Gordon et al. (2000) American journal of pathology 156(2):607-19; Laconi et al. Cell transplantation 2008;17(12):1415-21)

The right and left medial hepatic lobe and the left lateral hepatic lobe were removed, all other lobes stayed *in situ.* Animals were randomized into two groups. Group 1 received 2 x 10⁶ SC in 200 µl DMEM injected directly into the remaining right lateral hepatic lobe, group 2 received 200 µl DMEM instead. The entrance hole was closed by a short bipolar electro impulse. After thorough rinsing to remove remaining blood clots an osmotic pump (ALZET) was implanted into the abdominal cavity for continuous release of cyclosporine. The abdominal wall was closed in 3 steps by sutures only. The excised liver lobes were fixed in formalin for histological staging and analysis. Postoperative care was administered with buprenorphin and carprofen every 12 h and if needed.

### Blood levels

Blood was drawn through the tail vein every 7 days, starting from day 3 postoperatively to determine levels of cholinesterase, total protein, albumine, GOT, GPT, ferrum and lactate dehydrogenase.

### Example 6: Results

### Example 6.1: Flow cytometry

Human adipose stem cells (hADSC) were positive for the cell surface markers CD13, CD29, CD49a, CD63, CD73, CD90, CD105 and CD166. hADSC were negative for the cell surface markers CD31, CD34, CD44, CD45 and CD106. The cells therefore met the minimal consensus criteria for mesenchymal stem cells; see Figure 1.

### Example 6.2: Cell isolation and culture

### Adipogenic differentiation

Adipogenically induced cells showed a statistically significant higher oil red concentration than not induced controls in all donors; see Figure 2.

### Osteogenic differentiation

Osteogenically differentiated hADSC showed high extracellular calcium deposition, analyzed with alizarin red stain; see Figure 3. Non induced cells did not show extracellular calcium deposition.

### Example 6.3: Laboratory parameters

Prior to the first application of retrorsine, all blood levels taken from the experimental animals were consistent with the published base levels in literature.

### Example 6.4: Postoperative blood levels

Data were expressed as mean ± SE. Results were analyzed by the Student's *t* test and Mann Whitney Rank Sum test when normality test failed. P < 0.05 was considered statistically significant; see Figure 4.

### Albumin

Postoperative albumin levels were consistently under standard values. There were statistically significant higher albumin levels in cell treated animals than in non cell treated control animals in week one and two post surgery (p = 0,016-0,017). Albumin works as a good marker for liver synthesis.

### Total protein

Total protein was higher in cell treated animals throughout the whole period of analysis. Standard values were reached after the third week post surgery in cell treated animals and after the 6th week in control animals. Total protein levels were statistically significantly higher in the cell treated group compared to the control group in week one and two post surgery (p = 0,015-0,031).

### Choline esterase (CHE)

Postoperative CHE levels were clearly under standard values as a liver specific marker for significantly reduced liver function and synthesis, initially between 30 and 50 %. Standard value was not reached within the period of investigation. There was no statistically significant difference between cell treated and non cell treated groups. The non-treated control group did have higher CHE levels than the cell treated group. In both groups CHE levels slowly started to rise 6 weeks post surgery up to 75 % of standard value at week 10 post surgery.

### Lactate dehydrogenase (LDH)

Postoperative LDH levels in control animals lied consistently under standard values. In cell treated animals there was a LDH maximum in week 2 post surgery. In week 2, 4 end 5, LDH was statistically significantly higher in the cell treated group (p = 0,003-0,04).

### Glutamic oxaloacetic transaminase (GOT)

GOT lied higher in cell treated animals than in non cell treated animals from the first to the eighth week post surgery, in week 1 and 8 statistically significantly (p < 0,001-0,014).

### Glutamic pyruvic transaminaise (GPT)

Administration of allylalcohol and retrorsine led to an elevation of GPT levels before surgery in comparison to standard levels. Postoperative GPT levels were higher in the non cell treated group with a maximum within the second week post surgery. There was no statistically significant difference between cell treated and non cell treated animals.

GPT is considered to be liver specific.

### Alkaline phosphatase {AP}

Alkaline phosphatase was clearly elevated in comparison to standard levels post surgery until week 6. After week 6, alkaline phosphatase levels were higher in cell treated animals compared with control animals. There was no statistical significant difference (p > 0.05).

### Iron

Postoperative iron levels were clearly under standard levels. From week 3, postoperative animals treated with cells showed lower iron levels than control animals. There was no statistically significant difference in both groups (p > 0.05).

### Example 6.5: Histology

Cells migrated after injection from the center of the lobule to the periphery. SPIO-labeled cells were stained with Berliner Blau dye. No SPIO particles were found in macrophages. Due to the high iron intensity of the liver, SPIO particles could not be detected by MRI.

A high variance in the degree of necrosis from rat to rat, lobe to lobe and section to section could be observed. Tumor formation has not been observed over the whole follow up period of 16 weeks.

## Claims

1. A composition comprising human adipose stem cells for use in a method for the regeneration of liver, comprising administering said stem cells to the liver by intrahepatic infusion or injection, wherein the adipose stem cells are not embedded into a matrix consisting of a polymer, selected from a biopolymer and a synthetic polymer, forming a three-dimensional gel.

2. The composition for the use of claim 1 for the prevention, amelioration or treatment of acute or chronic liver disease.

3. The composition for the use of claim 2, wherein the acute or chronic liver disease is selected from loss of liver function, ischemia, fibrosis and/or cirrhosis.

4. The composition for the use of claim 2 or 3, wherein the liver disease is selected from the group consisting of: Liver ischemia, liver fibrosis, liver cirrhosis, acute liver failure, alcohol liver disease, Alpha-1-antitrypsin deficiency, autoimmune hepatitis, chronic hepatitis, cirrhosis, cholestatic liver disease, cystic disease of the liver, fatty liver, galactosemia, gallstones, Gilbert's syndrome, hemochromatosis, hepatitis A, hepatitis B, hepatitis C, liver cancer, neonatal hepatitis, non-alcoholic liver disease, non-alcoholic steatohepatitis, porphyria, primary biliary cirrhosis, primary sclerosing cholangitis, Reye's syndrome, sarcoidosis, steatohepatitis, tyrosinemia, type I glycogen storage disease, viral hepatitis, Wilson's disease, or any combination thereof.

5. The composition for the use of any of the preceding claims, wherein the adipose stem cell is isolated from human adipose tissue.

6. The composition for the use of any of the preceding claims, wherein the adipose stem cell is an autologous, heterologous or xenologous adipose stem cell.

7. The composition for the use of any of the preceding claims, wherein the adipose stem cell is a mesenchymal stem cell.

8. The composition for the use of any of the preceding claims, wherein the mesenchymal stem cell is positive for the cell surface markers CD13, CD29, CD49a, CD63, CD73, CD90, CD105 and/or CD166 and/or negative for the cell surface markers CD31, CD34, CD44, CD45 and/or CD106.

9. The composition for the use of any of the preceding claims, wherein the adipose stem cell is an undifferentiated mesenchymal stem cell, a differentiated mesenchymal stem cell or a genetically modified mesenchymal stem cell.

10. The composition for the use of any of the preceding claims, wherein the differentiated mesenchymal stem cell is an adipogenically, osteogenically or hepatogenically differentiated mesenchymal stem cell.

11. The composition for the use of any of the preceding claims, wherein the composition is formulated as a solution, suspension, dispersion or emulsion.

12. The composition for the use of the preceding claims, further comprising a biomaterial, on which the cells are coated.

13. The composition for the use of claim 12, wherein the biomaterial is fibronectin, fibrin, fibrinogen, thrombin, collagen, proteoglycans, chitosane, alginate and hyaluronic acid alginate, chitosane, hyaluronic acid, or polyethylene glycol.

14. The composition for the use of any of the preceding claims, further comprising serum or plasma.

15. The composition for the use of claim 14, wherein the serum is human serum or human serum from platelet-poor plasma.

16. The composition for the use of any of the preceding claims, wherein the adipose stem cell is administered (i) directly after isolation, (ii) after cultivation and/or proliferation in cell culture or (iii) after cryopreservation.

17. The composition for the use of any of the preceding claims, wherein the total protein level and/or the albumin level in the serum is increased, in comparison to a non-treated subject.

18. The composition for the use of any of the preceding claims, wherein the iron level in the serum is decreased, in comparison to a non-treated subject.

19. The composition for the use of any of the preceding claims, wherein the adipose stem cells can be detected for at least 4, 6, or preferably 8 weeks after infusion or transplantation.

## Patentansprüche

1. Eine Zusammensetzung umfassend menschliche Fettgewebestammzellen zur Verwendung in einem Verfahren zur Regeneration der Leber, umfassend das Verabreichen der Stammzellen in die Leber durch intrahepatische Infusion oder Injektion, wobei die Fettgewebestammzelle nicht in einem Polymer, ausgewählt aus einem Biopolymer und einem synthetischen Polymer, das ein dreidimensionales Gel bildet, eingebettet ist.

2. Die Zusammensetzung zur Verwendung nach Anspruch 1 zur Prävention, Linderung oder Behandlung einer akuten oder chronischen Leberkrankheit.

3. Die Zusammensetzung zur Verwendung nach Anspruch 2, wobei die akute oder chronische Leberkrankheit ausgewählt ist aus dem Verlust der Leberfunktion, Ischämie, Fibrose und/oder Zirrhose.

4. Die Zusammensetzung zur Verwendung nach Anspruch 2 oder 3, wobei die Leberkrankheit ausgewählt ist aus der Gruppe bestehend aus Leberischämie, Leberfibrose, Leberzirrhose, akutem Leberversagen, Alkoholleber, Alpha-1-Antitrypsin-Mangel, Autoimmunhepatitis, chronischer Hepatitis, Zirrhose, cholestatischer Lebererkrankung, zystischer Erkrankung der Leber, Fettleber, Galaktosämie, Gallenstein, Morbus Meulengracht, Hämochromatose, Hepatitis A, Hepatitis B, Hepatitis C, Leberkrebs, Neonatale Hepatitis, nichtalkoholische Leberkrankheit, nichtalkoholische Steatohepatitis, Porphyrie, primäre biliäre Zirrhose, primär sklerosierende Cholangitis, Reye Syndrom, Sarkoidose, Steatohepatitis, Tyrosinämie Typ 1 Glykogen - Speicherkrankheit, virale Hepatitis, Morbus Wilson, oder eine Kombination davon.

5. Die Zusammensetzung zur Verwendung nach einem der vorherigen Ansprüche, wobei die Fettgewebestammzelle aus humanem Fettgewebe isoliert ist.

6. Die Zusammensetzung zur Verwendung nach einem der vorherigen Ansprüche, wobei die Fettgewebestammzelle eine autologe, eine heterologe oder xenologe Fettgewebestammzelle ist.

7. Die Zusammensetzung zur Verwendung nach einem der vorherigen Ansprüche, wobei die Fettgewebestammzelle eine mesenchymale Stammzelle ist.

8. Die Zusammensetzung zur Verwendung nach einem der vorherigen Ansprüche, wobei die mesenchymale Stammzelle positiv für die Zelloberflächenmarker CD 13, CD 29, CD 49a, CD 63, CD 73, CD 90, CD 105 und/oder CD 166 und/oder negativ für die Zelloberflächenmarker CD 31, CD 34, CD 44, CD 45 und/oder CD 106 ist.

9. Die Zusammensetzung zur Verwendung nach einem der vorherigen Ansprüche, wobei die Fettgewebestammzelle eine undifferenzierte mesenchymale Stammzelle, eine differenzierte mesenchymale Stammzelle oder eine genetisch modifizierte mesenchymale Stammzelle ist.

10. Die Zusammensetzung zur Verwendung nach einem der vorherigen Ansprüche, wobei die differenzierte mesenchymale Stammzelle eine adipogen, eine osteogen oder eine hepatogen differenzierte mesenchymale Stammzelle ist.

11. Die Zusammensetzung zur Verwendung nach einem der vorherigen Ansprüche, wobei die Zusammensetzung als Lösung, Suspension, Dispersion oder Emulsion formuliert ist.

12. Die Zusammensetzung zur Verwendung der vorherigen Ansprüche, ferner umfassend ein Biomaterial, auf dem die Zellen beschichtet sind.

13. Die Zusammensetzung zur Verwendung nach Anspruch 12, wobei das Biomaterial Fibronektin, Fibrin, Fibrinogen, Thrombin, Kollagen, Proteoglykane, Chitosan, Alginat und Hyaluronsäure, Alginat, Chitosan, Hyaluronsäure oder Polyethylenglycol ist.

14. Die Zusammensetzung zur Verwendung nach einem der vorherigen Ansprüche, ferner umfassend Serum oder Plasma.

15. Die Zusammensetzung zur nach Anspruch 14, wobei das Serum Humanserum oder Humanserum aus plättchenarmen Plasma ist.

16. Die Zusammensetzung zur Verwendung nach einem der vorherigen Ansprüche, wobei die Fettgewebestammzelle (i) unmittelbar nach der Isolierung, (ii) nach der Kultivierung und/oder Proliferation in Zellkultur oder (iii) nach der Kryokonservierung verabreicht wird.

17. Die Zusammensetzung zur Verwendung nach einem der vorherigen Ansprüche, wobei die gesamt Proteinmenge und/oder der Albuminspiegel im Serum erhöht ist, im Vergleich zu einem nicht behandelten Subjekt.

18. Die Zusammensetzung zur Verwendung nach einem der vorherigen Ansprüche, wobei der Eisenspiegel im Serum verringert ist im Vergleich zu einem nichtbehandelten Subjekt.

19. Die Zusammensetzung zur Verwendung nach einem der vorherigen Ansprüche, wobei die Fettgewebestammzellen zumindest 4, 6 oder vorzugsweise 8 Wochen nach der Infusion oder Transplantation nachgewiesen werden können.

## Revendications

1. Composition comprenant des cellules souches du tissu adipeux humaines destinée à être utilisée dans un procédé de régénération du foie, comprenant l'administration desdites cellules souches au foie par une perfusion ou injection intrahépatique, où les cellules souches du tissu adipeux ne sont pas incorporées dans une matrice consistant en un polymère, sélectionné parmi un biopolymère et un polymère synthétique, formant un gel tridimensionnel.

2. Composition destinée à être utilisée selon la revendication 1 pour la prévention, l'amélioration ou le traitement d'une maladie hépatique aiguë ou chronique.

3. Composition destinée à être utilisée selon la revendication 2, où la maladie hépatique aiguë ou chronique est sélectionnée parmi une perte de fonction hépatique, une ischémie, la fibrose et/ou la cirrhose.

4. Composition destinée à être utilisée selon la revendication 2 ou 3, où la maladie hépatique est sélectionnée dans le groupe consistant en : une ischémie hépatique, la fibrose hépatique, la cirrhose hépatique, l'insuffisance hépatique aiguë, une maladie alcoolique du foie, le déficit en alpha-1-antitrypsine, une hépatite auto-immune, une hépatite chronique, la cirrhose, une maladie hépatique cholestatique, une maladie kystique du foie, la stéatose hépatique, la galactosémie, les calculs biliaires, le syndrome de Gilbert, l'hémochromatose, l'hépatite A, l'hépatite B, l'hépatite C, le cancer du foie, l'hépatite néonatale, une maladie du foie non alcoolique, la stéatohépatite non alcoolique, la porphyrie, la cirrhose biliaire primitive, la cholangite sclérosante primitive, le syndrome de Reye, la sarcoïdose, la stéatohépatite, la tyrosinémie, la maladie du stockage du glycogène de type I, une hépatite virale, la maladie de Wilson, ou une quelconque combinaisons de celles-ci.

5. Composition destinée à être utilisée selon l'une quelconque des revendications précédentes, dans laquelle la cellule souche du tissu adipeux est isolée à partir de tissu adipeux humain.

6. Composition destinée à être utilisée selon l'une quelconque des revendications précédentes, dans laquelle la cellule souche du tissu adipeux est une cellule souche du tissu adipeux autologue, hétérologue ou xénologue.

7. Composition destinée à être utilisée selon l'une quelconque des revendications précédentes, dans laquelle la cellule souche du tissu adipeux est une cellule souche mésenchymateuse.

8. Composition destinée à être utilisée selon l'une quelconque des revendications précédentes, dans laquelle la cellule souche mésenchymateuse est positive pour les marqueurs de surface cellulaire CD13, CD29, CD49a, CD63, CD73, CD90, CD105 et/ou CD166 et/ou négative pour les marqueurs de surface cellulaire CD31, CD34, CD44, CD45 et/ou CD106.

9. Composition destinée à être utilisée selon l'une quelconque des revendications précédentes, dans laquelle la cellule souche du tissu adipeux est une cellule souche mésenchymateuse indifférenciée, une cellule souche mésenchymateuse différenciée ou une cellule souche mésenchymateuse génétiquement modifiée.

10. Composition destinée à être utilisée selon l'une quelconque des revendications précédentes, dans laquelle la cellule souche mésenchymateuse différenciée est une cellule souche mésenchymateuse ayant subi une différenciation adipogénique, ostéogénique ou hépatogénique.

11. Composition destinée à être utilisée selon l'une quelconque des revendications précédentes, où la composition est formulée en tant que solution, suspension, dispersion ou émulsion.

12. Composition destinée à être utilisée selon les revendications précédentes, comprenant en outre un biomatériau, sur lequel les cellules sont revêtues.

13. Composition destinée à être utilisée selon la revendication 12, dans laquelle le biomatériau est la fibronectine, la fibrine, le fibrinogène, la thrombine, le collagène, des protéoglycanes, le chitosane, l'alginate et l'acide hyaluronique, l'alginate, le chitosane, l'acide hyaluronique, ou le polyéthylène glycol.

14. Composition destinée à être utilisée selon l'une quelconque des revendications précédentes, comprenant en outre du sérum ou du plasma.

15. Composition destinée à être utilisée selon la revendication 14, dans laquelle le sérum est du sérum humain ou du sérum humain issu de plasma pauvre en plaquettes.

16. Composition destinée à être utilisée selon l'une quelconque des revendications précédentes, dans laquelle la cellule souche du tissu adipeux est administrée (i) directement après un isolement, (ii) après une mise en culture et/ou prolifération en culture cellulaire ou (iii) après une cryoconservation.

17. Composition destinée à être utilisée selon l'une quelconque des revendications précédentes, dans laquelle le taux des protéines totales et/ou le taux d'albumine dans le sérum est augmenté, par comparaison à un sujet non traité.

18. Composition destinée à être utilisée selon l'une quelconque des revendications précédentes, dans laquelle le taux de fer dans le sérum est réduit, par comparaison à un sujet non traité.

19. Composition destinée à être utilisée selon l'une quelconque des revendications précédentes, dans laquelle les cellules souches du tissu adipeux peuvent être détectées pendant au moins 4, 6, ou de préférence 8 semaines après une perfusion ou transplantation.
